# EUROPÄISCHE PATENTSCHRIFT

(11) **EP 1 457 222 B1**
(45) Veröffentlichungstag und Bekanntmachung des Hinweises auf die Patenterteilung: **20.07.2005**
(21) Anmeldenummer: 04009173.8
(22) Anmeldetag: 27.12.2001
(51) Int. Cl.: A61M 16/08, A61M 16/00

(54) **Verfahren zur Schalldämmung bei Beatmungsgeräten**
Method of reducing noise in respirators
Méthode d'insonorisation pour appareils respiratoires

(30) Priorität: 19.02.2001 DE 10107990
(43) Veröffentlichungstag der Anmeldung: 15.09.2004
(62) Teilanmeldung aus: 01130832.7
(73) Patentinhaber: Weinmann Geräte für Medizin GmbH & Co. KG, 22525 Hamburg (DE)
(72) Erfinder: Feldhahn, Karl-Andreas, 22761 Hamburg (DE); Paesch, Rainer, 25451 Quickborn (DE)
(74) Vertreter: Klickow, Hans Henning,Dr.-Ing.

(56) Entgegenhaltungen:
- WO-A-99/22793
- WO-A-99/47197
- DE-A- 19 731 355

## Beschreibung

Die Erfindung betrifft ein Verfahren zur Schalldämmung bei der Beatmung eines Patienten, bei dem mindestens ein Teil des Atemgases durch eine Dämpfungsbox hindurchgeleitet wird, die mindestens einen Strömungskanal aufweist, in dessen Bereich die Atemluft an einem herausnehmbaren schalldämmenden Material vorbeigeführt wird.

Ein derartiges Verfahren wird beispielsweise in der DE-OS 197 31 355 beschrieben. Der Innenraum der Dämpfungsbox wird dabei durch Strömungsleitelemente unterteilt, um einen im Hinblick auf die Geräuschdämmung optimierten Strömungsweg bereitzustellen. Gemäß dem Stand der Technik ist es üblich, in ein metallisches Gehäuse der Dämpfungsbox zur Verbesserung der Dämpfungswirkung Platten aus Schaumstoff einzukleben.

Die bekannten Verfahren werden üblicherweise ausschließlich individuell für einen bestimmten Patienten genutzt, da sich eine Desinfektion aufgrund der vorliegenden Bauart schwierig gestaltet beziehungsweise nicht durchführbar ist. Die bekannten Konstruktionen führen ebenfalls dazu, daß auch bei einer Individualanwendung die Betriebsdauer durch eine zunehmende Anreicherung von Keimen und Bakterien im Bereich des Schaumstoffmaterials begrenzt ist.

Aus der WO-A-99/22793 ist bereits eine Dämpfungsbox bekannt, die mit einem plattenartigen schalldämmenden Material versehen ist. Die Dämpfungsbox ist zur Verwendung im Bereich von Beatmungsgeräten vorgesehen.

In der WO-A-99/47197 wird ein Verfahren zur Desinfektion eines inneren Gehäuses eines Beatmungsgerätes beschrieben, in dessen Bereich ein Lüfter zur Atemgasförderung angeordnet ist und das im Lufteingangsbereich mit einem Filtermaterial versehen ist.

Aufgabe der vorliegenden Erfindung ist es daher, ein Verfahren der einleitend genannten Art derart zu verbessern, daß sowohl eine erhöhte Nutzungsflexibilität als auch eine verlängerte Betriebsdauer erreicht werden.

Diese Aufgabe wird erfindungsgemäß dadurch gelöst, daß das schalldämmende Material nach einer vorgebbaren Betriebsdauer aus der Dämpfungsbox entnommen wird, daß anschließend eine Desinfektion eines Gehäuses der Dämpfungsbox durchgeführt wird und daß in die Dämpfungsbox ein keimfreies schalldämmendes Material zur Wiederherstellung der Betriebsfähigkeit eingesetzt wird, und daß das schalldämmende Material durch Eigenelastizität innerhalb der Dämpfungsbox fixiert wird.

Durch die lose Anordnung des schalldämmenden Materials ist es möglich, eine Desinfektion des Gehäuses separat und somit im Hinblick auf die jeweiligen Materialeigenschaften optimiert durchzuführen. Aufgrund dieser materialspezifischen Desinfektion ist es möglich, eine wiederverwendbarkeit der Vorrichtung zu erreichen. Ebenfalls ist es möglich, bei nachlassenden elastischen Eigenschaften des schalldämmenden Materials lediglich das plattenartige lose schalldämmende Material durch neue Bauteile zu ersetzen und das in der Regel aus Metall bestehende Gerätegehäuse weiter zu verwenden. Eine derartige Verfahrensweise hat sowohl unter wirtschaftlichen als auch unter ökologischen Gesichtspunkten eine hohe Bedeutung. Durch die elastische Verspannung des schalldämmenden Materials innerhalb des Gehäuses werden separate Befestigungselemente vermieden, die zu einer erschwerten Montage sowie zu Komplikationen bei der Desinfektion führen könnten.

Unter Desinfektion werden hierbei auch eine Sterilisation oder andere Maßnahmen verstanden, die dafür geeignet sind, eine ausreichende Keimfreiheit zu erreichen.

Durch das erfindungsgemäße Verfahren können somit ökonomische und ökologische Vorteile miteinander kombiniert werden, darüber hinaus wird auch eine äußerst einfache Montierbarkeit sowie Demontierbarkeit erreicht. Unter Berücksichtigung der deutlich verlängerten Nutzungsdauer der Dämpfungsbox kann hierdurch pro Zeiteinheit der Betriebsfähigkeit eine erhebliche Kostensenkung realisiert werden.

Eine effektive Keimabtötung kann dadurch erfolgen, daß die Desinfektion mindestens zum Teil thermisch durchgeführt wird.

Bei der Verwendung von wärmeempfindlichen Schalldämmungsmaterialien ist auch daran gedacht, daß die Desinfektion mindestens zum Teil chemisch durchgeführt wird.

Eine andere Desinfektionsvariante besteht darin, daß die Desinfektion mindestens zum Teil durch Strahlungsbeaufschlagung durchgeführt wird.

Eine einfach Montierbarkeit und Demontierbarkeit wird dadurch unterstützt, daß das schalldämmende Material durch Eigenelastizität innerhalb der Dämpfungsbox fixiert wird.

Zur Optimierung einer Strömungsführung wird vorgeschlagen, daß ein Strömungsweg innerhalb der Dämpfungsbox durch herausnehmbare Strömungsleitelemente begrenzt wird.

Eine effektive Schalldämmung von Gebläsegeräuschen kann dadurch erfolgen, daß die Atemgasdurchleitung durch die Dämpfungsbox hindurch durch ein innerhalb der Dämpfungsbox angeordnetes Gebläse unterstützt wird.

Eine einfache Handhabung bei gleichzeitig hoher mechanischer Stabilität kann dadurch erreicht werden, daß das schalldämmende Material auf einen plattenartigen Träger aufgebracht ist.

Eine hohe Dämmwirkung wird dadurch unterstützt, daß das schalldämmende Material aus Schaumstoff ausgebildet ist.

Eine weitere Erleichterung der Handhabung kann dadurch erreicht werden, daß mindestens ein Teil des schalldämmenden Materials als ein Einsatzteil für einen Gehäusedeckel der Dämpfungsbox ausgebildet ist.

Darüber hinaus erweist es sich als vorteilhaft, daß mindestens ein Teil des schalldämmenden Materials als ein Auskleidungsteil für ein Gehäuseunterteil der Dämpfungsbox ausgebildet ist.

Eine einfache Fertigung der verwendeten Bauteile kann dadurch unterstützt werden, daß das Auskleidungsteil einteilig als Faltbauelement mit Falzungen ausgebildet ist.

Eine geringe Anzahl der verwendeten Bauelemente kann dadurch erreicht werden, daß das Auskleidungsteil ein Zentralelement sowie Seitenklappen aufweist.

In der Zeichnung sind Ausführungsbeispiele der Erfindung schematisch dargestellt. Es zeigen:
- Fig. 1: eine Seitenansicht des Gehäuses mit abgenommenem Deckel,
- Fig. 2: eine Seitenansicht des Gehäuses gemäß Blickrichtung II in Fig. 1,
- Fig. 3: eine Draufsicht auf den Deckel,
- Fig. 4: eine Seitenansicht des Deckels gemäß Blickrichtung IV in Fig. 3,
- Fig. 5: eine Seitenansicht des Deckels gemäß Blickrichtung V in Fig.3,
- Fig. 6: eine Darstellung des Schaumstoffeinsatzes für den Deckel,
- Fig. 7: eine Darstellung des als Faltelement ausgebildeten Schaumstoffteils für das Gehäuseunterteil,
- Fig. 8: einen Querschnitt durch das Schaumstoffteil zur Veranschaulichung des Aufbaues aus einem Trägermaterial und einem schalldämmenden Material,
- Fig. 9: eine perspektivische Darstellung eines Strömungsleitelementes zum Einsetzen in die Dämpfungsbox,
- Fig. 10: eine perspektivische Darstellung des Strömungsleitelementes gemäß Fig. 9 bei einer Ansicht von hinten,
- Fig. 11: einen Blick in eine Dämpfungsbox mit abgenommenen Deckel und mit eingesetzten Strömungsleitelementen sowie eingesetztem Motorrahmen für das Gebläse,
- Fig. 12: einen Querschnitt gemäß Schnittlinie XII XII in Fig. 11,
- Fig. 13: eine perspektivische Darstellung des schalldämmenden Materials in Fig. 7 mit hochgeklappten Seitenteilen, um ein Einsetzen in das Gehäuseunterteil zu ermöglichen und
- Fig. 14: eine perspektivische Darstellung des schalldämmenden Materials gemäß Fig. 6.

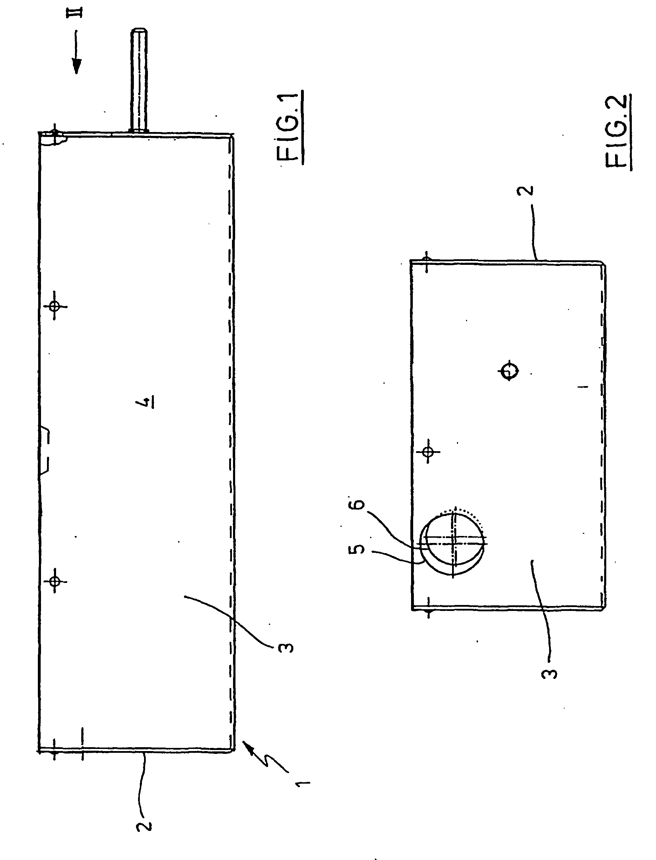

Figur 1 zeigt eine Seitenansicht einer Dämpfungsbox (1), wobei exakt ein Gehäuseunterteil (2) dargestellt ist. Das Gehäuseunterteil (2) weist Wandungen (3) auf, die vorzugsweise aus Metall ausgebildet sind. Ein von der Dämpfungsbox (1) umschlossener Innenraum (4) kann beispielsweise quaderförmig begrenzt sein.

Figur 2 zeigt eine Seitenansicht des Gehäuseunterteils (2) gemäß Figur 1. Es ist erkennbar, daß im Bereich der dargestellten Wandung ein Atemgaseinlaß (5) angeordnet ist. Durch den Innenraum (4) hindurch ist ein Atemgasauslaß (6) erkennbar.

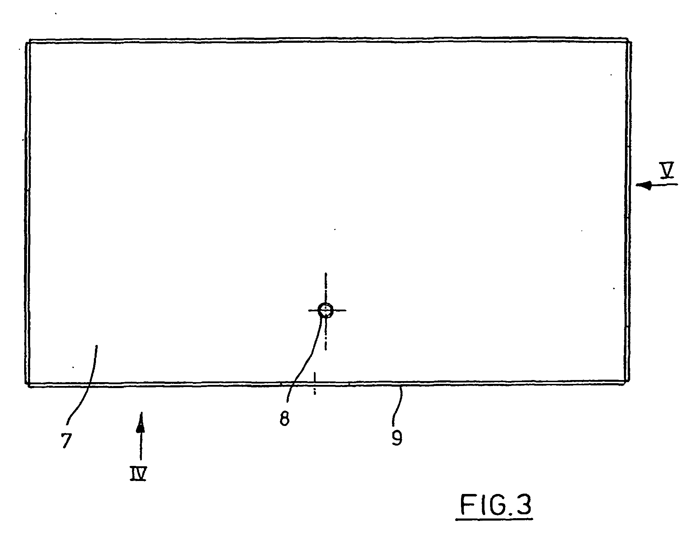

Figur 3 zeigt eine Draufsicht auf einen Gehäusedeckel (7), der auf das Gehäuseunterteil (2) aufsetzbar ist.

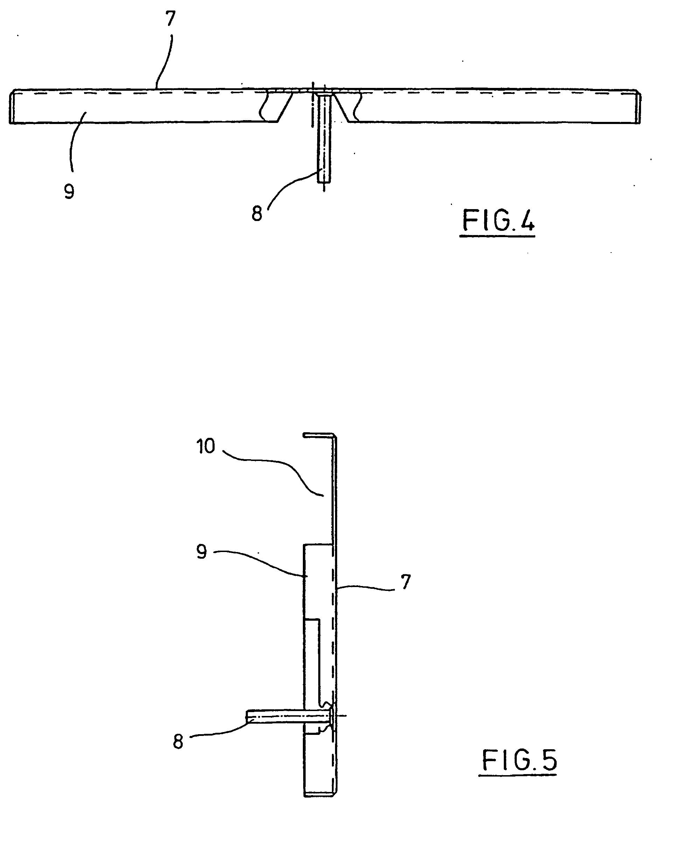

Figur 4 veranschaulicht in einer Seitenansicht die Gestaltung des Gehäusedeckels (7). Insbesondere ist erkennbar, daß der Gehäusedeckel (7) mit einem Halterungsbolzen (8) versehen ist, der für eine Halterung und Fixierung von zusätzlichen Bauteilen im Innenraum (4) dienen kann.

Die Seitenansicht des Gehäusedeckels (7) in Fig. 5 veranschaulicht, daß im Bereich einer seitlichen Deckelwandung (9) eine Ausnehmung (10) angeordnete ist.

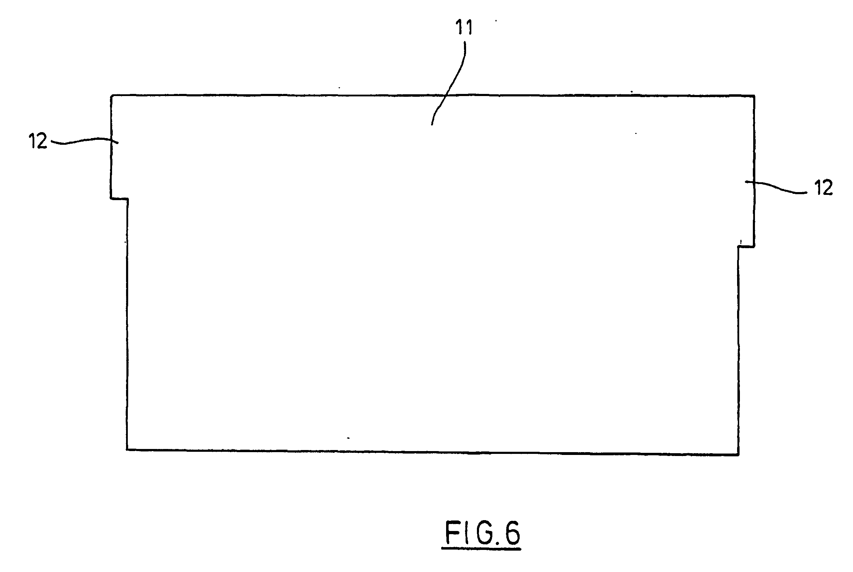

Fig. 6 zeigt ein Einsatzteil (11) aus einem schalldämmenden Material, das derart dimensioniert ist, daß ein Einsetzen in den Gehäusedeckel (7) möglich ist. Das Einsatzteil (11) weist Einsatzlaschen (12) auf, die in die Ausnehmungen (10) der Deckelwandung (9) einführbar sind. Nach einem Einsetzen des Einsatzteiles (11) in den Gehäusedeckel (7) begrenzen die Deckelwandungen (9) seitlich das Einsatzteil (11).

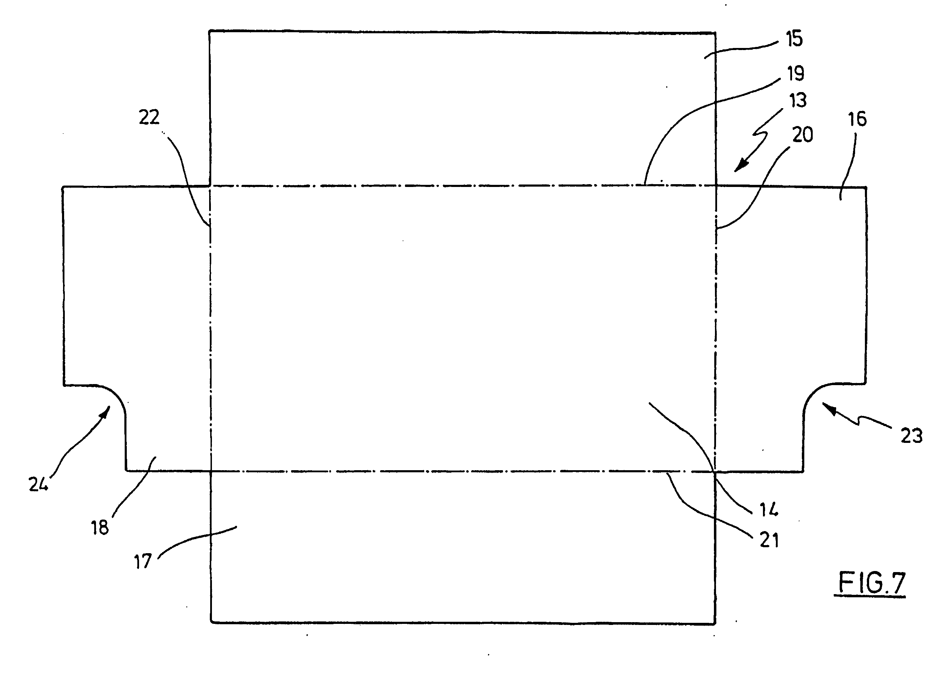

Fig. 7 zeigt ein Auskleidungsteil (13) für das Gehäuseunterteil (2). Das Auskleidungsteil (13) besteht aus einem Zentralelement (14) sowie Seitenklappen (15, 16, 17, 18), die über Falzungen (19, 20, 21, 22) mit dem Zentralelement (14) verbunden sind. Zwei der Seitenklappen (15, 16, 17, 18) sind mit Ausnehmungen (23, 24) versehen, die nach einem Einsetzen des Auskleidungsteiles (13) in das Gehäuseunterteil (2) eine Zuleitung und Ableitung von Atemgas durch den Atemgaseinlaß (5) sowie den Atemgasauslaß (6) hindurch ermöglichen.

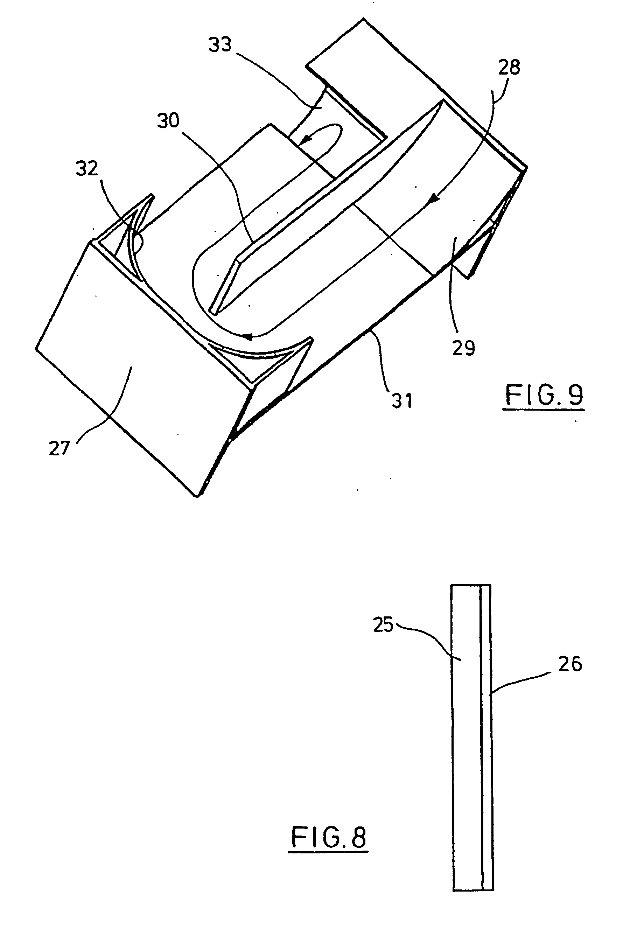

Fig. 8 zeigt eine Seitenansicht des Einsatzteiles (11) beziehungsweise des Auskleidungsteiles (13). Es ist erkennbar, daß ein schalldämmendes Material (25) von einem Träger (26) gehaltert ist. Eine Realisierung kann beispielsweise derart erfolgen, daß der Träger (26) aus Kunststoff, beispielsweise aus Polyethylen ausgebildet ist und daß als schalldämmendes Material (25) ein Schaumstoff verwendet wird. Grundsätzlich sind aber auch andere Materialpaarungen denkbar.

Eine Anwendung des Verfahrens und der Vorrichtung kann beispielsweise im Zusammenhang mit Geräten für die CPAP-Therapie oder im Zusammenhang mit anderen Schlaftherapien erfolgen. Ein grundsätzlicher Erfindungsgedanke ist darin zu sehen, daß die unmittelbar luftführenden Teile austauschbar ausgebildet werden.

In den Innenraum (4) können separate Strömungsleitelemente eingesetzt werden, um eine Unterteilung des Innenraumes (4) zur Optimierung der Strömungsführung zu erreichen. Die Strömungsleitelemente können beispielsweise aus einem festen Trägerteil aus Kunststoff ausgebildet sein, auf dem schalldämmendes Material, beispielsweise Schaumstoff fixiert ist. Die Strömungsleitelemente stehen vorzugsweise lose und herausnehmbar innerhalb des Innenraumes (4) und werden lediglich durch Formschluß oder elastische Verspannungen fixiert.

Fig. 9 zeigt eine perspektivische Darstellung eines Strömungsleitelementes (27), das zur Strömungsführung in den Innenraum (4) eingesetzt werden kann. Das Strömungsleitelement (27) besteht typischer Weise aus Kunststoff und kann zur Schalldämmung mit Schaumstoffteilen versehen werden. Es ist eine Strömungsführung (28) eingezeichnet, die veranschaulicht, daß im Bereich eines Eintrittes ein gerundetes Leitelement (29) angeordnet ist. Darüber hinaus weist das Strömungsleitelement (27) im wesentlichen senkrecht relativ zueinander angeordnete Unterteilungswände (30, 31) auf, die eine labyrinthartige Strömungsführung unterstützen. Insbesondere ist daran gedacht, im Bereich eines Überganges von Raumbereichen, die jeweils durch die Unterteilungen (30, 31) voneinander getrennt sind, gerundete Leitelemente (32, 33) anzuordnen. Vorzugsweise ist das Strömungsleitelement (27) mit einer quaderartigen Außenkontur zu versehen.

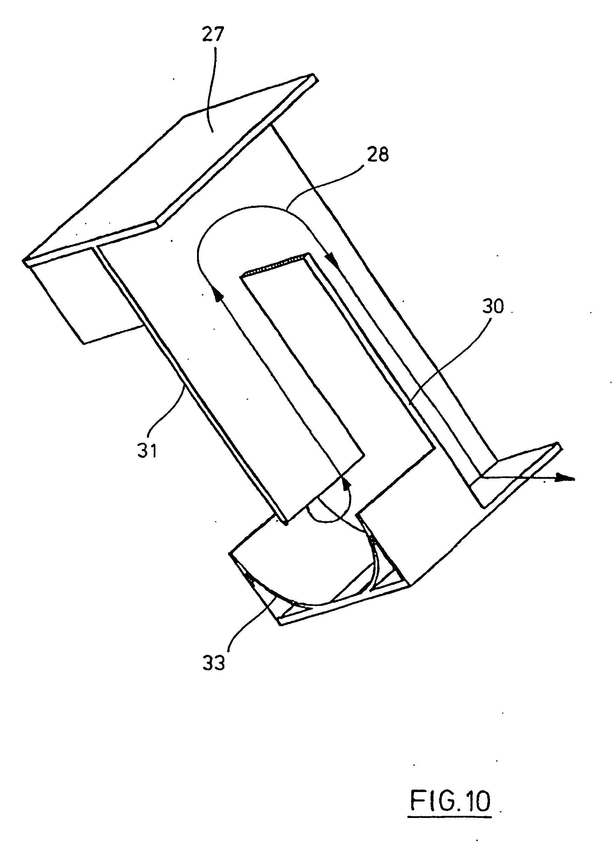

Fig. 10 veranschaulicht die Gestaltung des Strömungsleitelementes (27) im Bereich eines Strömungsaustrittes. Im Bereich dieses Strömungsaustrittes kann auf gerundete Leitelemente verzichtet werden, da eine Überleitung zum Gebläse erfolgt.

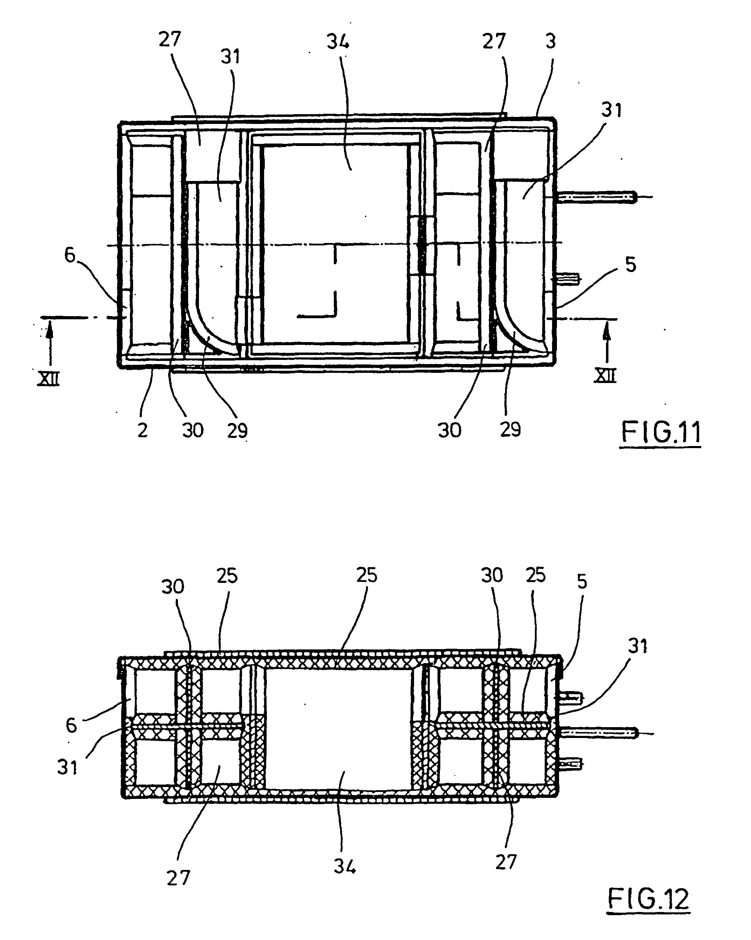

Fig. 11 zeigt das Gehäuseunterteil (2) mit zwei eingesetzten Strömungsleitelementen (27) sowie einem Motorrahmen (34), der zur Aufnahme des Gebläses vorgesehen ist.

Aus der Querschnittdarstellung in Fig. 12 ist erkennbar, wie durch die Strömungsleitelemente (27) eine labyrinthartige Strömungsführung bereitgestellt wird. Insbesondere ist auch erkennbar, wie die Überleitung der Atemgasströmung im den Bereich des Motorrahmens (34) erfolgt. Darüber hinaus ist zu erkennen, daß die in Fig. 9 und Fig. 10 dargestellten Wandungen des Strömungsleitelementes (27) mit schalldämmendem Material (25) versehen sind.

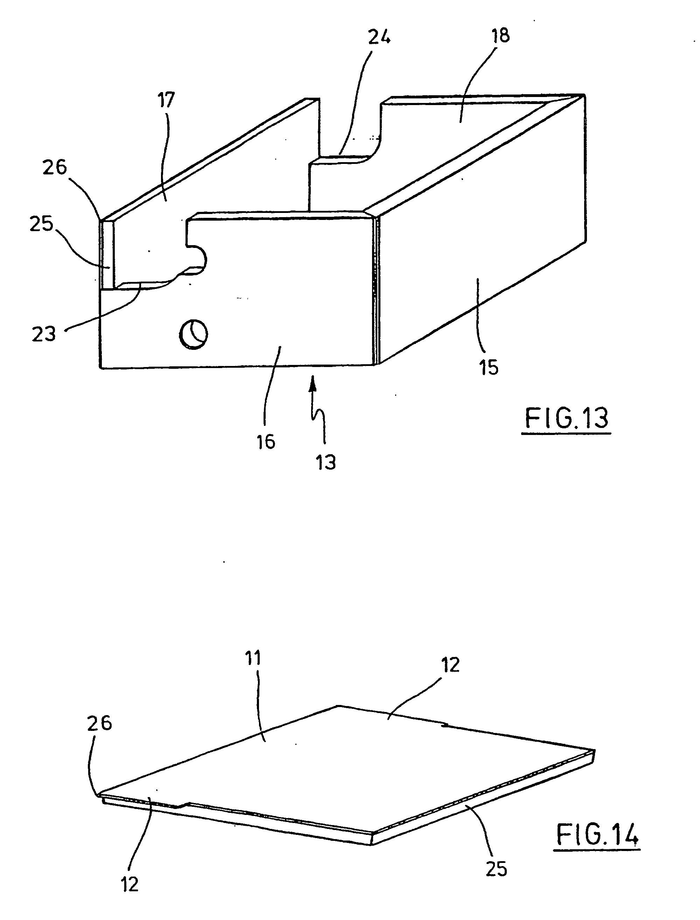

Fig. 13 zeigt das Auskleidungsteil (13) gemäß Fig. 7 nach einem Hochschwenken der Seitenklappen (15, 16, 17, 18), um ein Einfügen des Auskleidungsteiles (13) in das Gehäuseunterteil (2) zu ermöglichen. Insbesondere ist erkennbar, daß die plattenartigen Träger (26) außenseitig und daß das schalldämmende Material (25) innenseitig angeordnet sind.

Fig. 14 zeigt das Einsatzteil (11) in einer perspektivischen Darstellung. Auch hier ist erkennbar, daß in der Zeichenebene der plattenartige Träger (26) oben und das schalldämmende Material (25) unten angeordnet sind. Dies führt dazu, daß nach einem Einfügen des Einsatzteiles (11) in den Gehäusedeckel (7) der plattenartige Träger (26) am Gehäusedeckel (7) anliegt und daß das schalldämmende Material (25) dem Innenraum (4) zugewandt ist.

## Patentansprüche

1. Verfahren zur Schalldämmung bei der Beatmung eines Patienten, bei dem mindestens ein Teil des Atemgases durch eine Dämpfungsbox (1) hindurchgeleitet wird, die mindestens einen Strömungskanal aufweist, in dessen Bereich die Atemluft an einem herausnehmbaren schalldämmenden Material vorbeigeführt wird, **dadurch gekennzeichnet, daß** das schalldämmende Material nach einer vorgebbaren Betriebsdauer aus der Dämpfungsbox (1) entnommen wird, daß anschließend eine Desinfektion eines Gehäuses der Dämpfungsbox (1) durchgeführt wird, daß in die Dämpfungsbox (1) ein keimfreies schalldämmendes Material (25) zur Wiederherstellung der Betriebsfähigkeit eingesetzt wird und daß das schalldämmende Material (25) durch Eigenelastizität innerhalb der Dämpfungsbox (1) fixiert wird.

2. Verfahren nach Anspruch 1, **dadurch gekennzeichnet, daß** die Desinfektion mindestens zum Teil thermisch durchgeführt wird.

3. Verfahren nach Anspruch 1, **dadurch gekennzeichnet, daß** die Desinfektion mindestens zum Teil chemisch durchgeführt wird.

4. Verfahren nach Anspruch 1, **dadurch gekennzeichnet, daß** die Desinfektion mindestens zum Teil durch Strahlungsbeaufschlagung durchgeführt wird.

5. Verfahren nach einem der Ansprüche 1 bis 4, **dadurch gekennzeichnet, daß** ein Strömungsweg innerhalb der Dämpfungsbox (1) durch herausnehmbare Strömungsleitelemente begrenzt wird.

6. verfahren nach einem der Ansprüche 1 bis 5, **dadurch gekennzeichnet, daß** die Atemgasdurchleitung durch die Dämpfungsbox (1) hindurch durch ein innerhalb der Dämpfungsbox (1) angeordnetes Gebläse unterstützt wird.

## Claims

1. Method of noise reduction during the ventilation of a patient, in which method at least some of the respiratory gas is passed through a sound-muffling box (1) that has at least one flow channel in whose vicinity the respiratory air is fed past a removable sound-reducing material, **characterized in that** the sound-reducing material is removed from the muffling box (1) after a specifiable operating time, **in that** a casing of the muffling box (1) is then disinfected, **in that** a sterile sound-reducing material (25) is inserted into the muffling box (1) to restore the serviceability and **in that** the sound-reducing material (25) is held in position inside the muffling box (1) by its inherent elasticity.

2. Method according to Claim 1, **characterized in that** the disinfection is at least partly performed thermally.

3. Method according to Claim 1, **characterized in that** the disinfection is at least partly performed chemically.

4. Method according to Claim 1, **characterized in that** the disinfection is at least partly performed by irradiation.

5. Method according to any one of Claims 1 to 4, **characterized in that** a flow path inside the muffling box (1) is constrained by removable flow-conducting elements.

6. Method according to any one of Claims 1 to 5, **characterized in that** the passage of respiratory gas through the muffling box (1) is promoted by a fan disposed inside the muffling box (1).

## Revendications

1. Procédé d'insonorisation lors de la respiration artificielle d'un patient, dans lequel au moins une partie du gaz de respiration est conduit à travers un boîtier d'amortissement(1) qui présente au moins un canal d'écoulement dans la région duquel l'air de respiration est amené à contourner un matériau d'insonorisation amovible, **caractérisé en ce que** le matériau d'insonorisation est retiré du boîtier d'amortissement (1) après une durée de fonctionnement préétablie, **en ce que** l'on effectue ensuite une désinfection d'un logement du boîtier d'amortissement (1), **en ce que** l'on installe dans le boîtier d'amortissement (1) un matériau d'insonorisation (25) stérile pour rétablir la fonctionnalité, et **en ce que** le matériau d'insonorisation (25) est fixé à l'intérieur du boîtier d'amortissement (1) grâce à son élasticité propre.

2. Procédé selon la revendication 1, **caractérisé en ce que** la désinfection est réalisée au moins partiellement par voie thermique.

3. Procédé selon la revendication 1, **caractérisé en ce que** la désinfection est réalisée au moins partiellement par voie chimique.

4. Procédé selon la revendication 1, **caractérisé en ce que** la désinfection est réalisée au moins partiellement par application d'un rayonnement.

5. Procédé selon l'une des revendications 1 à 4, **caractérisé en ce qu'**un chemin de circulation à l'intérieur du boîtier d'amortissement (1) est délimité par des éléments amovibles de guidage de la circulation.

6. Procédé selon l'une des revendications 1 à 5, **caractérisé en ce que** le passage du gaz de respiration à travers le boîtier d'amortissement (1) est entretenu par une soufflante agencée à l'intérieur du boîtier d'amortissement (1).
